(19) Europäisches Patentamt
European Patent Office
Office européen des brevets

(11) Publication number: 0 376 511 A2

# (12) EUROPEAN PATENT APPLICATION

(21) Application number: 89312655.7

(51) Int. Cl.[5]: A61K 7/42, C08K 5/07

(22) Date of filing: 05.12.89

(30) Priority: 15.12.88 US 284539

(43) Date of publication of application:
04.07.90 Bulletin 90/27

(84) Designated Contracting States:
AT BE CH DE ES FR GB GR IT LI LU NL SE

(71) Applicant: ICI AMERICAS INC.
Concord Pike & New Murphy Road
Wilmington Delaware 19897(US)

(72) Inventor: Gosciniak, Donald John
1411 Manorwood Drive
West Chester Pennsylvania 19382(US)
Inventor: Neilan, James Patrick
25 Little Mill Lane
Bear Delaware 19701(US)

(74) Representative: James, David Gomer et al
Imperial Chemical Industries PLC Legal
Department: Patents Po Box 6 Bessemer
Road
Welwyn Garden City Herts, AL7 1HD(GB)

## (54) Ultraviolet radiation absorbing compositions of bis-1,3-diketone derivatives of benzene.

(57) Sunscreen compositions are described which contain certain bis-1,3-diketone derivatives of benzene when incorporated in a carrier in amounts ranging from 0.l-50% by weight.

EP 0 376 511 A2

## ULTRAVIOLET RADIATION ABSORBING COMPOSITIONS OF BIS-1,3-DIKETONE DERIVATIVES OF BENZENE

The present invention is directed to ultraviolet absorbing compositions comprising certain conjugated bis-1,3-diketone derivatives of benzene and blends thereof which are useful as protective coatings and to a method for protecting substrates against the harmful effects of actinic radiation. It is further directed to a process for making ultraviolet absorbing coating compositions.

Ultraviolet radiation absorbing coatings are useful in protecting substrates such as plastics against accelerated deterioration and the skin of warm blooded animals against severe erythema, edema and blistering when exposed to sunlight. The bis-diketone compositions of this invention are generally referred to as sunscreen compositions and blends thereof can be incorporated with waxes, oils, lacquers and soft resins in the preparation of furniture and auto polishes, as well as cosmetics, suntan oils, lotions, lipstick, hair treatments, skin formulations and in addition can be incorporated with contact lenses.

This invention relates to sunscreen compositions comprising a carrier having incorporated therein an effective amount of a ultraviolet absorber selected from a compound of general Formula I:

$RCOCH_2CO\text{-}A\text{-}COCH_2COR^1$ (I)

wherein -R and $R^1$ are independently selected from both linear and branched alkyl groups having from 1 to 10 carbon atoms or from $CR^2R^3Ar$ where $R^2$ and $R^3$ are selected from H and alkyl groups having 1-4 carbon atoms and Ar is a phenyl or an alkyl substituted phenyl group. The group -A- is selected form a bivalent 1,3 or 1,4 benzene radical having Formula II or III.

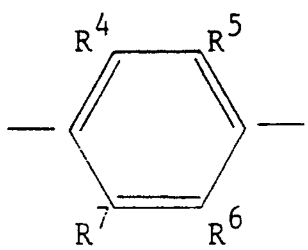

II

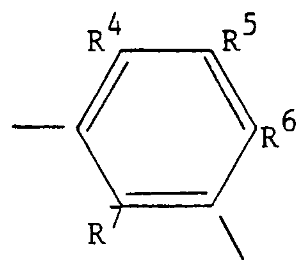

III

The substituents $R^4$, $R^5$, $R^6$, $R^7$ are independently selected from H or alkyl groups having from 1 to 10 carbon atoms or alkoxy groups having from 1 to 10 carbon atoms, providing that only one of the groups bonded to the aromatic ring is an alkoxy group. Preferred compounds are those where $R^1$ and R are either tert-butyl or isopropyl and $R^4$, $R^5$, $R^6$, $R^7$ are hydrogen.

The method for protecting substrates comprise topically applying the compound of formula I in an acceptable carrier. Of particular interest are compounds which provide selective absorption of UV radiation in the 290-320 nm as well as the 320-400 nm range of wave lengths. The compounds may be dissolved in the coating compositions or present as a finely divided solid or as a solid dispersed in an acceptable carrier. The selection of carrier used in the coating composition must not interfere with the absorption in the 290-400 nm range. In some instances interaction of the bis-diketone with a carrier shifts absorption outside the desired range and is not acceptable.

The compositions of the invention comprise UV filter compounds of Formula I in amounts needed to provide desired protection against the harmful effects of ultraviolet radiation. The concentration of the compounds in the composition is regulated such that when the composition is topically applied, the desired protection is provided. The amount needed to provide the desired protection can vary with the characteristics of the compound, that is, its extinction coefficient or substantively, the nature of the carrier, the source and intensity of the radiation and other well recognized variables. Suitable amounts can be readily determined by standard methods of testing. Preferably UV filter compounds are incorporated in an amount ranging from about 0.1 percent to about 50 percent by weight and usually in amounts of 1.0-30 percent by weight and preferred amounts ranging from 1.5-15 percent by weight based on the total weight of the coating composition.

Acceptable carriers include any vehicle or medium capable of incorporating the UV filter compound in a

manner permitting uniform topical application. The term "pharmaceutically acceptable" is intended as a qualifier when the carrier is dermatologically innocuous to warm blooded animals and cosmetically acceptable. However all carriers are not useful on skin. The carrier may comprise a wax, oil or cream base material in which the agent can be held in a clear solution or a uniform dispersion for example as submicron sized particles. Preferably the carrier comprises a suitable solvent or a mixture of solvents capable of dissolving the UV filter compounds to provide a concentration that is effective as a filtering agent when incorporated in the sunscreen formulation. Solvents which may be useful include alcohols, ketones, esters, polyol esters, oils, hydrocarbons, chlorinated hydrocarbons, ethers, polyethers, polyetherpolyols and other special solvents such as dimethylsulfoxide, dimethylformamide, dimethylisosorbide, isopropylmyristate and the like. Such solvents are considered useful only if they do not permanently interact with the active UV filtering compound of the invention to shift the total effective absorption outside the 290-400 nm range. Some of the above named ingredients are not pharmaceutically acceptable but are useful in other applications.

The invention is directed to method for protecting a substrate against the effects of ultraviolet radiation which comprises topically applying the above described compounds in a carrier.

The sunscreening compositions may be applied as a clear liquid or a lotion comprising a water-in-oil, oil-in-water or a multiple emulsion. Either the oil or water base or both may be used as a carrier for the sunscreening composition. The oil base material and the water and oil base compositions will form a continuous film of the UV filtering compound. Such films also provide long lasting protection against sun induced erythema. Sunscreening formulations are generally used in hot weather and at beaches where people enjoy bathing activity. It is also essential that the protective coating applied to the skin is not appreciably affected by water or perspiration. The pharmaceutically acceptable compositions herein disclosed are included in a thin layer protective coating on the skin of warm blooded animals and provide long lasting protection against erythema and do not appreciable decompose over practical periods of exposure to sunlight.

In general the compounds are synthesized by condensing the enolate of the corresponding ketone with a diester such as dimethyl terephthalate in an inert solvent such as tetrahydrofuran or n-butylether.

The following compounds exemplify, but do not limit, the active compounds of the Formula I:

$RCOCH_2CO\text{-}A\text{-}COCH_2COR^1$

1,1′-(1,4-phenylene)-bis-4,4-dimethylpentane-1,3-dione
1,1′-(1,3-phenylene)-bis-4,4-dimethylpentane-1,3-dione
1,1′-(1,4-phenylene)-bis-4-methylpentane-1,3-dione
1,1′-(1,4-phenylene)-bis-6-methylheptane-1,3-dione
1,1′-(1,4-phenylene)-bis-pentane-1,3-dione
1,1′-(1,4-phenylene)-bis-5-methylhexane-1,3-dione
1,1′-(2,3,5-trimethyl-1,4-phenylene)-bis-4-methyl pentane-1,3-dione
1,1′-(2,3,5,6-tetramethyl-1,4-phenylene)-bis-4-methyl pentane-1,3-dione
1,1′-(2-ethoxy-3,5-dimethyl-1,4-phenylene)-bis-4-methylpentane-1,3-dione
1,1′-(3-butyl-1,4-phenylene)-bis-4,4-dimethylpentane-1,3-dione
1,1′-(1,4-phenylene)-bis-4-phenylbutane-1,3-dione

Compounds wherein R and $R^1$ are alkyl groups can be prepared by reacting an aromatic diester with the appropriate ketone enolate in an inert solvent. The substituted aromatic diesters can be prepared according to known methods, such as that described by Wals et al. (Rec Trav. Chim. Pays-Bas 87 p. 65 1968). Those compounds wherein R and $R^1$ are $CR^2R^3Ar$ can be prepared by reacting the corresponding diacetyl benzene with the appropriate ester of phenyl acetic acid or a substituted phenyl acetic acid.

The following preparative examples serve as nonlimiting illustrations of the type of compounds included in the invention and all parts and percentages are expressed on a weight basis unless otherwise specified.

## Preparation 1

### Synthesis of 1,1′-(1,4-phenylene)-bis-4,4-dimethylpentane-1,3-dione.

In a 500 ml three neck flask was placed 150 ml of dry tetrahydrofuran under nitrogen. To this was added 7.8 g (0.2 mol) sodium amide. Pinacolone (10 g, 0.1 mol) was then added dropwise over 15 minutes and then stirred for an additional 15 minutes. Dimethylterepthalate (9.7 g, 0.05 mol) was then added and the mixture heated to reflux for 4 hours. The contents were then cooled to room temperature and poured into

cold water. The pH was then adjusted to 5.2 with hydrochloric acid. The resulting precipitate was collected by filtration and washed with water. Drying of the solids resulted in isolation of 14.2 g material, 91% pure by spectrophotometric analysis. Recrystallization from methanol resulted in pure material (mp = 124-126 °C) having a peak absorption of 342 nm; molar extinction coefficient = 32, 340.

Preparation 2.

Synthesis of 1,1′-(1,3-phenylene)-bis-4,4-dimethylpentane-1,3-dione.

The procedure of preparation 1 was repeated using dimethyl isophthalate in place of the terephthlate ester. This material has a maximum absorption of 314 nm and a molar extinction coefficient of 30360.

Preparation 3.

Synthesis of 1,1′-(1,4-phenylene)-bis-4-methylpentane-1,3-dione.

This material is prepared by repeating the procedure of preparation 1 substituting methyl isopropyl ketone for pinacolone in equivalent molar amounts. The product has a peak absorption of 343 nm and a molar extinction coefficient of 32,313.

Preparation 4.

Synthesis of 1,1′-(1,4-phenylene)-bis-6-methylheptane-1,3-dione.

According to the procedure outlined for preparation 1, methyl isoamyl ketone is substituted for pinacolone in equivalent stoichiometric amounts. The peak absorption for the product is 344 nm and the molar extinction coefficient is 31,556.

Preparation 5.

Synthesis of 1,1′-(1,4-phenylene)-bis-pentane-1,3-dione.

The procedure of preparation 1 is repeated using methyl ethyl ketone in place of pinacolone in stoichiometric amounts. The product has a peak absorption at 341 nm and a molar extinction coefficient of 28,122.

Preparation 6

Synthesis of 1,1′-(1,4-phenylene)-bis-5-methylhexane-1,3-dione.

This material is prepared by repeating preparation 1 employing stoichiometric amounts of methyl isobutyl ketone in place of pinacolone. This solid has a maximum absorption peak at 343 nm and a molar extinction coefficient of 33.000.

Preparation 7

Synthesis of 1,1′-(2,3,5,6-tetramethyl-1,4-phenylene)-bis-4-methylpentane-1,3-dione.

To a suspension of 0.96g NaH (97% active) in 50 ml dry THF is added dropwise 1.72 g methyl isopropyl ketone. The reaction is stirred at ambient temperature for 30 minutes, followed by addition of 2.78g 1,4-dicarboethoxy-2,3,5,6-tetramethylbenzene over a 2 minute period. The reaction is then heated to reflux for 5 hours, cooled to room temperature and poured into 300 ml ice water. The pH is adjusted to 2-3 with concentrated HCl and the product isolated by filtration.

Preparation 8

Synthesis of 1,1′-(1,4-phneylene)-bis-4-phenylbutane-1,3-dione.

To a suspension of 1.92g NaH (97% active) in 50 ml dry THF is added 3.2 g 1,4-diacetylbenzene in 50 ml THF. The reaction is stirred at ambient temperature for 30 minutes, followed by addition of 5,92g methyl phenylacetate over 5 minutes. The reaction is heated to reflux for five hours, cooled to room temperature, and poured into 300 ml ice water. The pH is adjusted to 2-3 with conecntrated HCl and the product isolated by filtration.

It has been established that actinic radiation between 290nm and 320nm produces substantially all the burning or erythemal energy and a substantial portion of the tanning energy, while the radiation between 320nm and 400nm produces incident tanning. The cosmetic industry has divided these spectra into the burning range UV-B (290-320nm) and the tanning range UV-A (320-400nm). Since approximately 76% of the physiological tanning potential of sunlight is found in the UV-B range and the balance is found in the UV-A range, it is desirable to have a substantial amount of the radiation in those ranges filtered out before it produces a harmful effect on the surface of human skin. While sunscreen lotions have been formulated to be most effective in the UV-B range recent studies have indicated that it is desirable to have collective adsorption in the UV-A range as well. It has been difficult to find a practical compound which effectively absorbs in both ranges. Therefore, formulators must resort to the combination of two compounds which are each effective either in the UV-B, or UV-A range to provide maximum skin protection. No single compound falling within the definition of formula I is effective over the entire 290-400nm range and therefore two or more compounds can be selected and blended within the formulation at varying concentrations until the desired balance between burning and tanning is accommodated. Such a combination is shown in Example 13. It is preferred to have a formulation having at least one compound which absorbs in the 290-320nm range and at least one other which absorbs in the 320-400nm range. At least one is selected from Formula I.

The use of the UV filters of the invention can be demonstrated in lotion formulations which are topically applied to the surface of the skin. The effectiveness of the UV light absorbers are tested on human subjects by treating a 1 cm square section of a subjects' back with predetermined amounts of lotion, exposing the treated areas to UV light for a period of time and thereafter making a visual comparison with untreated and fully masked skin areas. The SPF (skin protection factor) is calculated by comparing the effects of radiation on protected skin with the unprotected skin.

Besides the SPF determinations on humans, many in vitro methods and in vivo tests on animal models are also widely used. Some of these methods yield results which correlate well with SPF determined on humans and are useful tools for evaluating new compounds.

The following lotions and creams will serve to illustrate but not limit those which can be used in the practice of the invention.

In general, typical formulating techniques are well known to skilled formulators and usually require that the filtering agent be first added to the oil phase which is thereafter emulsified. With regards to examples 1-4 all ingredients can be mixed together and stirred in conventional apparatus. Since in many cases a single compound used at a reasonable concentration does not effectively protect throughout the whole region of the earth reaching solar UV spectrum, blends of two or more UV absorbers can be used in a formulation to afford greater protection. To illustrate the effectiveness of the compounds of the invention in sunscreen formulations Preparation 3 was formulated into creams and lotions for extensive testing. The formulations are shown in Table 1.

Sunscreen Formulas

Table 1

| Ingredient | Examples (% by Weight) | | | |
|---|---|---|---|---|
| (A) | (1) | (2) | (3) | (4) |
| Compound of Prep. 3 | 5 | 2 | 5 | 2 |
| Mineral oil (Carnation) | 5 | 5 | 0 | 0 |
| Stearyl alcohol | .5 | .5 | 0 | 0 |
| Cetyl alcohol | .5 | .5 | 0 | 0 |
| Silicone oil (SF-96,350 cs) | .5 | .5 | 0 | 0 |
| Polyoxyethylene (21) stearyl ether | 2 | 2 | 0 | 0 |
| Polyoxyethylene (2) stearyl ether | 2 | 2 | 0 | 0 |
| (B) | | | | |
| Water (deionized) | 73.95 | 76.95 | 0 | 0 |
| Carbopol® 934, (2% soln) | 10 | 10 | 0 | 0 |
| (C) | | | | |
| Sodium hydroxide (10% aq.) | .2 | .2 | 0 | 0 |
| (D) | | | | |
| DNDNH-55 (Glyco) | .35 | .35 | 0 | 0 |
| (E) | | | | |
| Dimethyl isosorbide | 0 | 0 | 95 | 98 |

Blending Procedure for Examples 1 and 2

Blend ingredients A and heat to 70° C. In a separate container heat ingredients B to 75° C and add to A. Add C to AB then cool to 40° C. Add ingredient D with stirring.

Procedure Examples 3 and 4

Simple solutions of E were made for SPF testing as described below.

The solution of Example 3 was applied to 8 specimens of excised hairless mouse epidermis at a level of 1 mg/cm$^2$. The epidermis was exposed to ultraviolet radiation in the UV-B and UV-A range and compared with unprotected skin similarly exposed. Average test results for SPF are listed in Table 2.

Table 2

| Peak Absorption | Mol Extinct Coefficient | SPF | | Standard Deviation | |
|---|---|---|---|---|---|
| (nm) | Mol Wt | UV-B | UV-A | UV-B | UV-A |
| 344 .26 | 117.8 | 1.4 | 1.43 | .35 | |
| Control | | | | | |
| 100% DMI | | <1.0 | <1.0 | 0 | 0 |

In addition to their use in coating skin surfaces to prevent sunburn the compositions of the invention can also be employed in various formulations such as waxes, oils, lacquers and soft resins in the preparation of furniture and auto polishes, cosmetics, lipstick, hair treatments, skin formulations and contact lenses. The compounds of the invention act as filtering agents and may be used singly or in combination to provide a wider range of protection. The following formulations are given to demonstrate a few of the many applications.

| Example No. | Filtering Agent | Carrier Ingredients | Composition (% by Wt) |
|---|---|---|---|
| 5 | Aerosol Hairdressing | | |
| | Prep 4 | | 5.0 |
| | | Decaglycerol monolaurate | 2.0 |
| | | Polypropylene (200) monooleate | 3.0 |
| | | Ethoxylated (10) lanolin alcohols | 1.0 |
| | | Propylene glycol | 2.0 |
| | | Ethyl alcohol, anhydrous | 39.5 |
| | | Protein polypeptide (20% alcoholic) | 1.2 |
| | | Isopropyl myristate | 1.3 |
| | | Propellant 11 | 15.0 |
| | | Propellant 12 | 30.0 |
| | | Water | q.s. |

Procedure for Formula: Dissolve all ingredients in slightly warmed ethylalcohol, avoiding loss of the alcohol, add the water, and agitate well to disperse any haze. Filter the concentrate and fill into aerosol containers. Add propellants.

| Example No. | Filtering Agent | Carrier Ingredients | Composition (% by Wt) |
|---|---|---|---|
| 6 | Formula for Creamy Type Lipstick Base | | |
| | Prep 6 | | 5 |
| | | Carnauba wax | 3 |
| | | Candelilla wax | 7 |
| | | Ozokerite® | 3 |
| | | Beeswax | 7 |
| | | Lanolin | 10 |
| | | Castor oil | 60 |
| | | Isopropyl myristate | 5 |
| | | Perfume | q.s. |
| 7 | Water-In-Oil (W/O), Detergent Resistant, Liquid Auto Polish | | |
| | Part A | 2.00% Durmont® 500 Montan Wax | (Dura Commodities) |
| | Part B | 0.75% DC 530 Silicone Fluid | (Dow Corning) |
| | | 4.25% DC 531® Silicone Fluid | |

1.50% SPAN® 80 (ICI Americas) sorbitan monooleate
10.00% Kerosene
16.50% Stoddard Solvent
5.0% Preparation 5

| Example No. | Filtering Agent | Carrier Ingredients | Composition (% by Wt) |
|---|---|---|---|
| | Part C | 10.00% Kaopolite™ SFO | (Kaopolite) |
| | Part D | 50.00% Water | |

Method of Preparation:

1. Melt wax in Part A (85-90°C)
2. Add Part B ingredients to melted wax and stir to blend well. Return temperature to 85-90°C.
3. Add Part C to Part A/Part B blend and mix until uniform with medium agitation. Keep temperature in the 85-90°C range.
4. Heat Part D to 95°C and slowly add to the blend with high speed stirring until emulsion is obtained.
5. Cool to 40-45°C with continuous stirring.
6. Homogenize.

| Example No. | Filtering Agent | Carrier Ingredients | Composition (% by Wt) |
|---|---|---|---|
| 8 | Neutral Base Lacquer | | |

| Materials | Pounds |
|---|---|
| Urethane 60% N.V. | 32 |
| Long oil alkyd 60% N.V. | 352 |
| Triton X-45 | 7.5 |
| Nuxtra® Calcium 6% | 12 |
| Bentone Jell 8% | 28 |
| Disperse the bentone jell under high speed cowles and add: | |
| Preparation 1 | 16 |
| Low odor mineral spirits | 85 |
| Cyclodex cobalt 6% | 3 |
| JK 270-70% | 76 |
| Water | 205 |
| Anti skin | 2 |

| | |
|---|---|
| Viscosity: | 80-85 KU |
| W/G: | 7.84 |
| 60° Gloss: | 85 |
| SAG: | 6 ml |

9 O/W Paraffin Wax Emulsion

Part A 50% Paraffin wax

5% SPAN 60/TWEEN 60 (50/50) (ICI Americas) (sorbitan monostearate/20 dendro sorbitan monostearate)
5% Preparation 6

Part B 40% Water

Method of Preparation:

1. Melt Part A ingredients together and heat to 80°C.
2. Heat Part B to 85°C.
3. Add Part B to Part A slowly with moderate agitation until inversion occurs. Add remaining water rapidly.
4. Cool in cold water bath with slow agitation to approximately 35°C.

10 O/W Soft Microcrystalline Wax Emulsion

Part A 30% Microcrystalline wax (Ultraflex Amber Wax-Petrolite Corp.)
30% SPAN® 60/TWEEN® 60 (78/22)
5% Preparation 2

| Example No. | Filtering Agent | Carrier Ingredients | Composition (% by Wt) |
|---|---|---|---|
| | Part B | 62% Water | |

Method of Preparation:

1. Melt together Part A ingredients and heat to 80-90°C.
2. Heat Part B to boiling.
3. Add Part B to Part A slowly with moderate agitation until inversion occurs. Add remaining water rapidly.
4. Remove from heat and cool to room temperature without stirring.

11 O/W Carnauba Wax Emulsion

| | | |
|---|---|---|
| Part A | 10% Carnauba wax | |
| | 3% TWEEN 80 (ICI Americas) (20 dendro sorbitan monooleate) | |
| | 5% Preparation 3 | |
| Part B | 82% Water | |

| Example No. | Filtering Agent | Carrier Ingredients | Composition (% by Wt) |
| --- | --- | --- | --- |

II (cont.)

Method of Preparation:

1. Melt Part A ingredients together and heat to 95°C and hold.
2. Heat Part B to boiling.
3. Add Part B to Part A slowly with moderately fast stirring until inversion occurs. Add remaining water rapidly.
4. Remove emulsion from heat and cool rapidly with stirring.

| SUNSCREEN LOTION | | |
| --- | --- | --- |
| Example 12 | | |
| PHASE | INGREDIENTS (SUPPLIERS) | PERCENT BY WEIGHT |
| A | Petrolatum, Snow White USP (Ruger) | 35.00 |
| | Brij® 721 (ICI Americas surfactant) | 1.16 |
| | Brij 72 (ICI Americas surfactant) | 3.86 |
| | Silicone Oil, 350cs (Ruger) | 3.00 |
| | Preparation 7 | 5.00 |
| | Uvinul® M-40 (BASF) | 3.00 |
| B | Water | 48.08 |
| | Carbopol® 934 (B. F. Goodrich) | 0.40 |
| C | Sodium Hydroxide (10% Aqueous Solution) | 0.40 |
| D | Dowicil® 200 (DOW) | 0.10 |
| Preparation: Heat (A) to 60° C. Heat (B) to 65° C. Add (B) to (A) slowly with moderate agitation. Add (C). Cool to 50° C. Add (D). Cool, while stirring to 35° C. | | |

| SUNSCREEN LOTION | | |
|---|---|---|
| Example 13 | | |
| PHASE | INGREDIENTS (SUPPLIERS) | PERCENT BY WEIGHT |
| A | Arlamol® E (ICI Americas, 30 dendro stearyl alcohol | 7.00 |
| | Stearyl Alcohol | 2.50 |
| | Silicone Oil, 350 cs (Ruger) | 5.00 |
| | Arlasolve 200 (ICI) | 2.10 |
| | Brij 72 (ICI) | 4.90 |
| | Preparation No 1 | 5.00 |
| | Preparation No 2 | 3.00 |
| B | Water | 70.00 |
| | Carbopol 934 (B. F. Goodrich) | 0.20 |
| C | Sodium Hydroxide (10% Aqueous Solution) | 0.20 |
| D | Dowicil 200 (DOW) | 0.10 |
| Preparation: Heat (A) to 65° C. Heat (B) to 70° C. Add (B) to (A) slowly with moderate agitation. Add (C). Cool to 50° C. Add (D). Cool, while stirring to 35° C. | | |

| SUNSCREEN LOTION | | |
|---|---|---|
| Example 14 | | |
| PHASE | INGREDIENTS (SUPPLIERS) | PERCENT BY WEIGHT |
| A | Arlamol E (ICI) | 7.00 |
| | Stearyl Alcohol | 2.50 |
| | Silicone Oil, 350cs (Rugher) | 5.00 |
| | Arlasolve® 200 (ICI 20 dendro isohexadecyl alcohol) | 2.10 |
| | Brij 72 (ICI) | 4.90 |
| | Preparation 7 | 8.00 |
| B | Water | 70.00 |
| | Carbopol® 934 (B. F. Goodrich) | 0.20 |
| C | Sodium Hydroxide (10% Aqueous Solution) | 0.20 |
| D | Dowicil 200 (DOW) | 0.10 |
| Preparation: Heat (A) to 60° C. Heat (B) to 65° C. Add (B) to (A) slowly with moderate agitation. Add (C). Cool to 50° C. Add (D). Cool, while stirring to 35° C. | | |

| SUNSCREEN LOTION | | |
|---|---|---|
| Example 15 | | |
| PHASE | INGREDIENTS (SUPPLIERS) | PERCENT BY WEIGHT |
| A | Petrolatum, Snow White USP (Ruger) | 35.00 |
| | Brij 721 (ICI) | 1.16 |
| | Brij 72 (ICI) | 3.86 |
| | Preparation 8 | 8.00 |
| | Silicone Oil, 350cs (Ruger) | 3.00 |
| B | Water | 49.08 |
| | Carbopol 934 (B. F. Goodrich) | 0.40 |
| C | Sodium Hydroxide (10% Aqueous Solution) | 0.40 |
| D | Dowicil 200 (DOW) | 0.10 |
| Preparation: Heat (A) to 60° C. Heat (B) to 65° C. Add (B) to (A) slowly with moderate agitation. Add (C). Cool to 50° C. Add (D). Cool, while stirring to 35° C. | | |

| SUNSCREEN LOTION | | |
|---|---|---|
| Example 16 | | |
| PHASE | INGREDIENTS (SUPPLIERS) | PERCENT BY WEIGHT |
| A | Arlamol E (ICI) | 7.00 |
| | Stearyl Alcohol | 2.50 |
| | Silicone Oil, 350 cs (Ruger) | 5.00 |
| | Arlasolve® 200 (ICI) | 2.10 |
| | Brij 72 (ICI) | 4.90 |
| | Preparation 5 | 5.50 |
| B | Water | 72.50 |
| | Carbopol 934 (B. F. Goodrich) | 0.20 |
| C | Sodium Hydroxide (10% Aqueous Solution) | 0.20 |
| D | Dowicil 200 (DOW) | 0.10 |
| Preparation: Heat (A) to 65° C. Heat (B) to 70° C. Add (B) to (A) slowly with moderate agitation. Add (C). Cool to 50° C. Add (D). Cool, while stirring to 35° C. | | |

## Claims

1. A sunscreen composition comprising an acceptable carrier containing a compound selected from Formula I:
$RCOCH_2CO\text{-}A\text{-}COCH_2COR^1$
wherein -R and $R^1$ are independently selected from both linear and branched alkyl groups having from 1 to 10 carbon atoms or from $CR^2R^3Ar$ where $R^2$ and $R^3$ are selected from H and alkyl groups having 1-4 carbon atoms and Ar is phenyl or alkyl substituted phenyl group, wherein -A- is selected from a bivalent 1,3 or 1,4 benzene radical having Formula II or III.

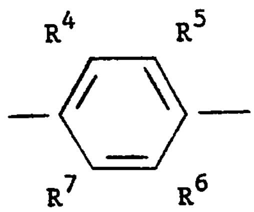

II

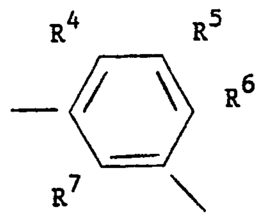

III

$R^4$, $R^5$, $R^6$, $R^7$ are independently selected from H or alkyl groups having from 1 to 10 carbon atoms or alkoxy groups having from 1 to 10 carbon atoms, providing that only one of the groups bonded to the aromatic ring is an alkoxy group.

2. A composition as claimed in claim 1 in which -A- is 1,4-phenylene and R and $R^1$ are both the same and are both tert-butyl, iso-propyl, iso-amyl, ethyl or iso-butyl.

3. A composition as claimed in claim 1 in which -A- is 1,3-phenylene and R and $R^1$ are both the same and are both isopropyl or tert-butyl.

4. A composition as claimed in claim 1 in which -A- is 2,3,5,6-tetramethyl-1,4-phenylene and R and $R^1$ are both the same and are both isopropyl.

5. A composition as claimed in claim 1 in which -A- is 1,4-phenylene and R and $R^1$ are both the same and are both benzyl.

6. A composition as claimed in any one of claims 1 to 5 wherein the compound of Formula I is incorporated in said composition in an amount ranging from about 0.1 to about 50% by weight.

7. A composition as claimed in claim 6 wherein the compound of Formula I is incorporated in said composition in an amount ranging from 1 to about 15%.

8. A composition as claimed in any one of claims 1 to 7 wherein the compound of Formula 1 is dissolved in the carrier.

9. A composition as claimed in any one of claims 1 to 7 wherein the carrier is an aqueous emulsion.

10. A composition as claimed in any one of claims 1 to 9 having at least one compound selected from Formula I and at least one other compound which absorbs radiation in the 290-400nm range.

11. A method for protecting a substrate from the effects of ultraviolet radiation which comprises topically applying to the substrate a composition as claimed in any one of claims 1 to 10.